# EUROPÄISCHE PATENTSCHRIFT

(11) **EP 2 785 265 B1**
(45) Veröffentlichungstag und Bekanntmachung des Hinweises auf die Patenterteilung: **23.09.2015**
(21) Anmeldenummer: 12797694.2
(22) Anmeldetag: 12.11.2012
(51) Int. Cl.: A61B 17/3207, A61B 17/22, A61B 17/221, A61M 25/00, A61M 25/10

(54) **VORRICHTUNG ZUM ABLÖSEN WANDSTÄNDIGER THROMBEN AUS EINEM KÖRPERGEFÄSS**
DEVICE FOR DETACHING PARIETAL THROMBI FROM A BLOOD VESSEL
DISPOSITIF POUR RETIRER DES THROMBUS PARIÉTAUX D'UN VAISSEAU DU CORPS

(30) Priorität: 30.11.2011 DE 102011120004
(43) Veröffentlichungstag der Anmeldung: 08.10.2014
(73) Patentinhaber: Universitätsklinikum Freiburg, 79104 Freiburg (DE)
(72) Erfinder: HEHRLEIN, Christoph, 79104 Freiburg (DE)
(74) Vertreter: Rösler, Uwe
(86) Internationale Anmeldenummer: PCT/EP2012/004694
(87) Internationale Veröffentlichungsnummer: WO 2013/079156

(56) Entgegenhaltungen:
- EP-A2- 0 385 920
- WO-A1-2010/115642
- WO-A2-2007/089897
- US-A1- 2005 021 071
- US-A1- 2005 283 166

## Beschreibung

### Technisches Gebiet

Die Erfindung bezieht sich auf eine Vorrichtung zum Ablösen wandständiger aus einem Körpergefäß mit einem Katheter, längs dessen Katheterlängserstreckung wenigstens ein Katheterabschnitt vorgesehen ist, dessen zuordenbarer Katheteraußendurchmesser durch ein Mittel veränderbar ist und in dessen zuordenbaren Katheterwand wenigstens eine die Katheterwand quer zur Katheterlängserstreckung vollständig durchsetzende Wandöffnung eingebracht ist.

### Stand der Technik

Zur interventionellen Therapie von durch gefäßwandständigen Thromben verengten und von Körperflüssigkeiten durchströmten Gefäßen ist eine Vielzahl von Katheterbasierten, medizinischen Instrumenten bekannt, die der schonenden Ablation von an Gefäßinnenwänden alterungs- oder krankheitsbedingten Ablagerungen dienen. Die häufig auch als Thrombektomie-Kathetersysteme bezeichneten medizinischen Instrumente, sehen jeweils eine zur Gewebeablation geeignete Katheterspitze vor, durch die ablatiertes Thrombus-Material zumeist unterstützt mittels eines innerhalb des Katheterlumens vorherrschenden Unterdruckes extrakorporal entsorgt wird.

Hierzu ist aus der US 2010/0087844 A1 ein mechanisches Thrombektomie-Kathetersystem mit einem mehrlumigen Katheter zu entnehmen, der mit Hilfe eines Führungs- und Positionierungsdrahtes relativ zu einer Thrombose positionierbar ist, die zunächst über einen Spülkanal mit einer Lyse-Lösung vorbehandelt wird. Über einen längs des Katheters verlaufenden Instrumentenkanal tritt anschließend ein korbförmig ausgebildetes Schabwerkzeug distalseitig aus dem Katheter und fragmentiert das im Wege von axialer Hin- und Herbewegung das umliegende Thrombus-Material. Über einen weiteren, längs des Katheters vorgesehenen Aspirationskanal können die separaten Thrombus-Materialfragmente gesammelt und extrakorporal verbracht werden.

Im Unterschied zu dem axial beweglichen, korbartig ausgeführten Schabwerkzeug, wie vorstehend erläutert, zeigen die US 2006/0074441 A1 sowie US 2011/0040314 A1 drahtförmig ausgebildete Thrombus-Material-Abtragewerkzeuge, die jeweils an einer drahtförmig ausgebildeten Werkzeugspitze eine sinusoidale Drahtform besitzen, die bei Rotation um die Drahtlängsachse die Wirkung einer spindelförmigen Schneidstruktur annimmt und bei Inkontakttreten mit Gefäßwandadhäsivem Thrombus-Material dieses aufgrund rotatorischer Scherkräfte zu zerkleinern und abzutragen vermag. Losgelöste Thrombus-Materialfragmente werden auch hier mit einem geeignet platzierten Aspirationskatheter extrakorporal verbracht.

Ein weiteres, Thrombus-Material ablatierendes Werkzeug ist in der US 2006/0064073 A1 beschrieben, das einen durch einen Aspirationskatheter geführten Werkzeugkatheter betrifft, an dessen distalen Katheterbereich ein radial zur Katheterachse keilförmig abspreizbares Ablationsmittel angebracht ist, das durch rotatorische Bewegung um die Katheterachse intravaskuläres Thrombus-Material abzutrennen und zu fragmentieren vermag. Durch die keilförmig ausgerichteten Werkzeugschenkel und der vermittels des Aspirationskatheters vorherrschenden Saugwirkung gelangen die fragmentierten Thrombosematerialpartikel proximalwärts zur Werkzeugkatheterspitze in den Aspirationskatheter. Eine vorteilhafte Ergänzung zu dem vorstehend beschriebenen mechanischen Thrombektomie-Kathetersystem ist in der US 2006/0253145 A1 beschrieben, in der am distalen Ende des Werkzeugkatheters ein inflatierbarer Ballon vorgesehen ist, der zum einen den Werkzeugkatheter axial innerhalb des Gefäßes und somit relativ zum abzutragenden Thrombus-Material zentriert und andererseits dafür sorgt, dass keine von der Gefäßwand separierte Fragmente von Thrombus-Material distalseits zum fächerförmig aufgeweiteten und rotierenden Abtragewerkzeug unkontrolliert in die der Blutbahn gelangen können.

Die US 2007/0208361 A1 beschreibt ein Atherektomie-Kathetersystem, das einen Aspirationskatheter vorsieht, der distalseitig von einem radial aufweitbaren Stentgeflecht sowie einer zentrisch geführten Nadel überragt ist, die gemeinsam mit rotierend gelagert sind und vergleichbar eines Bohrkopfes durch distalseitigen Vorschub längs eines stenösen Blutgefäßes Thrombus-Material punktiert, separiert und zerkleinert. Die fragmentierten Thrombus-Materialstücke werden über den Aspirationskatheter extrakorporal verbracht.

Eine weitere Art zur lokalen Abtrennung stenosierter Gewebebereiche stellen Kathetersysteme mit Schneidwerkzeugen dar. Aus der US 2009/0270800 A1 ist ein Katheter zu entnehmen, an dessen distalen peripheren Außenbereich in Umfangsrichtung einzelne Blähkörper angeordnet sind, um die axiale Lage der distalen Katheterspitze relativ zu einem zu behandelnden Gefäß individuell zu positionieren. Ferner ist innerhalb des Katheters eine über die Katheterspitze führbare Schneidklinge vorgesehen, mit deren Hilfe Thrombus-Material von der Gefäßwand abtrennbar ist. Abgetrennte Thrombus-Materialstücke gelangen anschließend über einen Aspirationskanal längs der Katheteranordnung.

Eine vergleichbare Katheteranordnung ist der WO 2010/132748 A1 zu entnehmen, die ebenfalls ein Schneidwerkzeug vorsieht, das sowohl axial durch eine radial aufspreizbare Katheterspitze als auch über eine seitlich längs der Katheteranordnung vorgesehene Öffnung führbar gelagert ist.

Aus der US 2008/0300532 A1 ist ein Fluid-durchströmter Thrombektomie-Katheter zu entnehmen, der mit Hilfe eines Führungsdrahtes längs einer stenösen Gefäßstelle positionierbar ist und längs eines distalen Katheterabschnittes über wenigstens zwei quer zur Katheterlängserstreckung orientierte Öffnungen verfügt, von denen eine Öffnung als Fluidaustrittsöffnung dient, aus der ein Fluidstrom quer zur Gefäßlängserstreckung auf das abzutragende Thrombus-Material auftrifft und dieses lokal von der Gefäßinnenwand unter Ausbildung kleinster Thrombus-Materialfragmente ablöst. Die abgelösten Thrombusfragmente gelangen über die zweite Öffnung in das Innere des Katheters, längs dem die mit Thrombus-Material durchsetzte Fluidströmung extrakorporal nach außen gelangt.

Schließlich beschreibt die DE 10 2009 017 050 A1 eine Vorrichtung zum Ablösen von Konkrementen aus einem Körpergefäß mit einem Katheter, durch den ein Werkzeugkatheter relativ zu einer abzutragenden Thrombose positionierbar ist. Der Werkzeugkatheter weist ein stentförmig ausgebildetes Fängerelement auf, das sich nach distalseitigem Ausbringen aus dem Katheter radial selbständig aufweitet. Das Fängerelement verfügt über als Längsschlitze ausgebildete Eintrittsöffnungen, durch die das zu entfernende Konkrementmaterial, insbesondere Thromben, in das Innere des Fängerelementes eindringen. Durch axiale sowie auch rotatorische Bewegungen des Werkzeugkatheters kommt es aufgrund auftretender Scherkräfte zum Ablösen des durch die Eintrittsöffnungen in das Innere des Fängerelementes ragenden Thrombus-Materials, das in Form fragmentierter Einzelstücke durch Applikation eines innerhalb des Werkzeugkatheters anliegenden Unterdruckes extrakorporal verbracht werden kann. Zur Entnahme der Katheteranordnung aus dem Gefäß wird das Fängerelement proximalwärts durch radial wirkende Kompressionskräfte in den Kanal gezogen und in diesem komprimierten Zustand extrakorporal entnommen. DE 10 2009 017050 A1 offenbart eine Vorrichtung entsprechend dem Oberbegriff des Anspruchs 1.

Alle vorstehend bekannten Lösungen stellen technisch zum Teil aufwendige Katheterausbildungen dar, deren operative Handhabung hohe Ansprüche an den Operateur stellen, zumal die zumeist scharfkantigen Werkzeuge zum lokalen intravasalen Abtrag wandständiger Thromben mit großer Vorsicht zu bedienen sind, um Verletzungen an Gefäßwänden zu vermeiden.

Die Druckschrift US 2010/0125239 A1 erläutert einen Katheter mit einem aufblasbaren Ballon, mit dem Medikamente an eine bestimmte Stelle innerhalb eines Lumens, z.B. einer Arterie, appliziert werden können. In einer Ausführungsvariante ist der Ballon von einer porösen Membran umgeben. Im Zwischenraum zwischen Ballon und Membran befindet sich ein Medikament. Durch Ansteuern von auf der Membran befindlichen Elektroden und die dabei zugeführte Wärmeenergie öffnen sich die Membranporen und Moleküle eines Medikaments werden durch den mit dem Ballon erzeugten Druck durch die Membranporen zum zu behandelnden Gewebe gedrückt. Der Ballon bewirkt zudem eine radiale Expansion, bei der der Katheter mit dem umgebenden Gewebe in Kontakt tritt.

Die in US 7 063 671 B2 offenbarten Vorrichtungen zur Entnahme von Proben bzw. Entfernung von Polypen aus einem Körper eines Patienten weisen Aktoren aus elektroaktiven Polymermaterial auf, die per elektrische Stimulation kontrahiert bzw. expandiert werden können. Durch entsprechende Betätigung der Aktoren können Öffnungen in der Vorrichtung erweitert werden, um eine Probe oder den Polyp zu umfassen.

### Darstellung der Erfindung

Der Erfindung liegt die Aufgabe zugrunde, eine Vorrichtung zum Ablösen gefäßwandständiger Thromben aus einem Körpergefäß möglichst einfach, kostengünstig und für den Operateur leicht bedienbar auszugestalten, so dass eine Gefäßwandverletzung weitgehend ausgeschlossen werden kann. Zudem gilt es, Gefäßwand-ständiges Thrombus-Material sicher und effizient, d.h. möglichst rückstandsfrei, von der Gefäßwand zu entfernen und die von der Gefäßwand separierten Thrombus-Fragmente sicher extrakorporal zu verbringen.

Die Lösung der der Erfindung zugrunde liegenden Aufgabe ist im Anspruch 1 angegeben. Merkmale, die die lösungsgemäße Vorrichtung in vorteilhafter Weise weiterbilden, sind Gegenstand der Unteransprüche sowie der weiteren Beschreibung, insbesondere unter Bezugnahme auf die Ausführungsbeispiele zu entnehmen.

Die lösungsgemäße Vorrichtung geht von einem nächstkommenden Stand der Technik aus, der in der vorstehend gewürdigten Druckschrift DE 10 2009 017 050 A1 beschrieben ist und eine Vorrichtung zum Ablösen Gefäßwand ständiger Thromben aus einem Körpergefäß mit einem Katheter zeigt, längs dessen Katheterlängserstreckung wenigstens ein Katheterabschnitt vorgesehen ist, in dessen zuordenbaren Katheterwand wenigstens eine die Katheterwand quer zur Katheterlängserstreckung vollständig durchsetzende Wandöffnung vorgesehen ist, sowie einem Mittel, mit dem ein dem Katheterabschnitt zuordenbarer Katheteraußendurchmesser veränderbar ist.
Die lösungsgemäße Vorrichtung zeichnet sich dadurch aus, dass die Katheterwand, längs des wenigstens einen Katheterabschnitts, längs dem die wenigstens eine Wandöffnung eingebracht ist, aus einem elastisch verformbaren Material besteht, vorzugsweise aus einem elastisch verformbaren Elastomer, das rohr- oder schlauchförmig ausgebildet ist und ein inneres Katheterlumen umfasst. Der aus einem elastisch verformbaren Material bestehende Katheter ist durch ein separates Mittel von einem Zustand mit relativ kleinerem Katheteraußendurchmesser in einen Zustand mit relativ größerem Katheteraußendurchmesser im Wege einer elastischen Formänderung überführbar, wobei das Mittel auf den über wenigstens eine Wandöffnung verfügenden Katheter einen äußeren Zwang ausübt. Das Mittel ist als ein in den wenigstens einen Katheterabschnitt einführbarer Verdrängungskörper ausgebildet. Fällt der von außen wirkende Zwang auf den Katheter weg, vorzugsweise durch Separation des Mittels vom Katheter bzw. Beseitigung der Wechselwirkung zwischen dem Mittel und dem Katheter, so nimmt der Katheter aufgrund materialinhärenter, elastischer Rückstellkräfte den Zustand des relativ kleineren Katheteraußendurchmessers selbständig ein.

Die wenigstens eine Wandöffnung weist vorzugsweise einen schlitzförmigen, runden, ovalen oder n-eckigen Öffnungsquerschnitt auf, der sich beim Überführen des Katheters vom Zustand mit dem relativ kleineren Katheteraußendurchmesser in den Zustand mit dem relativ größeren Katheteraußendurchmesser durch den äußeren Zwang aufweitet und im umgekehrten Fall selbständig verkleinert.

Die lösungsgemäße Kathetervorrichtung für einen lokalen, intravasalen Abtrag wandständiger Thromben nutzt das Wirkprinzip elastischer Rückstellkräfte, wie sie vergleichbar in einem zwanghaft radial aufgeweiteten Schlauchstück vorherrschen, das aus einem elastischen Elastomermaterial gefertigt ist. Wird zudem in der Schlauchwand eine lokale Wandöffnung eingebracht, beispielsweise in Form eines Einschnittes, so weitet sich die Schnittöffnung im Wege der radialen Aufweitung des Schlauchabschnittes auf. Zur Aufweitung dient bspw. ein in den Schlauch einführbarer Verdrängungskörper. Wird hingegen der Verdrängungskörper aus dem Schlauchabschnitt entfernt, so sorgen die innerhalb des Schlauchmaterials inhärenten elastischen Rückstellkräfte für eine selbstständige Durchmesserreduzierung in den Zustand der Ursprungsform sowie ein Verkleinern bzw. Wiederverschließen der aufgeweiteten Wandöffnung.

Wird im radial aufgeweiteten Zustand weiches Material, in Form von Thrombus-Material, in die geweitete Wandöffnung gebracht, so umschließt die Wandöffnung das in der Wandöffnung befindliche Thrombus-Material und klemmt dieses regelrecht fest ein, sobald der die radiale Schlauchdurchmesseraufweitung verursachende äußere Zwang wegfällt. Auf diese Weise ist es möglich, Gefäßwand-adhärentes Thrombus-Material portionsweise abzutragen und mit Hilfe einer lösungsgemäß ausgebildeten Katheteranordnung extrakorporal zu verbringen.

Eine einfachste Ausführungsform zur Realisierung des lösungsgemäßen Kathetersystems besteht somit aus einem, aus flexiblen, elastischen Wandmaterial bestehenden Katheter, in dessen Katheterwand zumindest im distalen Katheterbereich wenigstens eine Wandöffnung eingebracht ist. Die Wandöffnung kann in unterschiedlichster Ausbildungsform realisiert sein, so beispielsweise in Form eines bloßen Wandschnittes oder einer Wandöffnung, die im Wege eines Stanzvorganges oder mit Hilfe einer ähnlichen Materialabtragetechnik in die Katheterwand einbringbar ist. Die Wandöffnung kann grundsätzlich beliebige Öffnungsquerschnittsformen aufweisen, so beispielsweise runde oder von einer runden Querschnittsform abweichende Formen, wie beispielsweise n-eckige Querschnittsformen. Zur Aufweitung des Katheters dient ein Mandrin, der längs des inneren Lumens des Katheters einführbar ist und dessen Mandrinaußendurchmesser größer gewählt ist, als der Katheterinnendurchmesser im kräftefreien Zustand. Durch Einführen eines derartigen Mandrins in den Katheter wird der Katheter radial aufgeweitet. Der Mandrin sollte aus einem quer zur Mandrinlängsachse formstabilen Material sein, jedoch eine um die Mandrinlängsrichtung flexible Form aufweisen, um so den Mandrin längs gebogener Gefäßverläufe hindurchführen zu können.

Der Mandrin weist in einer einfachsten Ausführungsform eine glatte Oberfläche auf, die zugleich im Bereich einer aufgeweiteten Wandöffnung eine innere Begrenzungsfläche für das durch die Wandöffnungsweite und -tiefe bestimmte Aufnahmevolumen darstellt, in das das wandadhärente Thrombus-Material eindringen kann, um anschließend in der vorstehend erläuterten Weise portionsweise extrahiert werden zu können.

In einer bevorzugten Ausführungsvariante sieht der Mandrin an seiner Mandrinoberfläche konkave Ausnehmungen vor, die im Falle des in dem Katheter eingeführten Mandrins deckungsgleich zu den Wandöffnungen innerhalb der Katheterwand angeordnet sind. Form und Größe der konkaven Ausnehmungen entsprechen Form und Größe der Wandöffnungen oder sind größer als die Form und Größe der Wandöffnungen ausgebildet. Durch die das Aufnahmevolumen jeder einzelnen Wandöffnung, wie vorstehend beschrieben, einseitig begrenzende, nun konkav ausgebildete Begrenzungsfläche vergrößert sich das Aufnahmevolumen deutlich gegenüber dem vorstehend geschilderten Fall.

Besonders vorteilhaft ist die Verwendung eines hohl ausgebildeten Mandrins, der nun selbst über eine die Mandrinwand durchsetzenden Mandrinwandöffnungen verfügt, die, im Falle des in den Katheter eingeführten Hohlmandrins deckungsgleich zu den Wandöffnungen innerhalb der Katheterwand angeordnet sind. Auch in diesem Fall entsprechen Form und Größe der Mandrinwandöffnungen der Form und Größe der Wandöffnungen des Katheters oder sind größer als Wandöffnungen ausgebildet. Im Falle eines Hohlmandrins bietet es sich in besonders vorteilhafter Weise an, längs des Mandrins Unterdruck anzulegen, durch den der Abtrenn- und Entnahmevorgang des jeweils portionsweise zu ablatierenden Thrombus-Materials vereinfacht werden kann.

Alternativ zur Verwendung eines Mandrins, zu Zwecken der radialen Aufweitung des Katheters, ist es ebenso möglich, einen inflatierbaren Ballon vorzusehen, der in einem deflatierten Zustand durch den Katheters führbar ist und im Katheterbereich, in dem die Wandöffnungen vorgesehen sind, inflatiert wird, wodurch der Katheter zumindest in diesem Bereich radial aufgeweitet wird.

In beiden Fällen, nämlich der Verwendung eines Mandrins in all seinen Ausgestaltungsmöglichkeiten oder eines inflatierbaren Ballons, bewirkt die radiale Aufweitung des innerhalb eines mit Thrombus-Material zugesetzten Gefäßes geeignet positionierten Katheters eine Erhöhung des Anpressdruckes der Katheteraußenseite an wandständiges Thrombus-Material, wodurch das Thrombus-Material in die aufgeweiteten Wandöffnungen innerhalb der Katheterwand regelrecht hinein gepresst wird. Durch Entfernen des Mandrins aus oder durch Deflation des Ballons innerhalb des Katheterinneren zieht sich der Katheter aufgrund der materialinhärenten, elastischen Rückstellkräfte selbständig radial zusammen, wodurch das innerhalb der Wandöffnungen befindliche und wandadhärente Thrombus-Material durch im Bereich der Wandöffnungen wirkenden Scherkräfte zum einen in den Wandöffnungen festgehalten und zum anderen vom restlichen wandadhärenten Thrombus-Material abgetrennt wird.

Der Abtrennvorgang kann in einer bevorzugten Ausführungsform durch axiale und/oder radiale Relativbewegungen des Katheters relativ zur stenosierten Gefäßstelle unterstützt werden. Die Relativbewegungen können entweder manuell durch den Operateur vorgenommen oder mit Hilfe einer geeignet proximalseits am Katheter vorgesehenen Vibratoreinheit unterstützt werden.

Ferner sieht eine weitere vorteilhafte Ausführungsform den Anschluss einer Unterdruckquelle zumindest längs des Katheters vor, so dass der Abtrennvorgang des wandadhärenten Thrombus-Materials sowie eine nachfolgende sichere Entfernung des abgelösten Thrombus-Materials durch den Katheter proximalseitig hindurch unterstützt werden können.

Das lösungsgemäße Kathetersystem sieht keinerlei die gesunde Gefäßwand möglicherweise verletzenden Schneidwerkzeuge vor, die getrennt zum Katheter intravasal zu führen sind, wie dies im einschlägigen Stand der Technik der Fall ist. Vielmehr ermöglicht das lösungsgemäße Kathetersystem eine schonende und vollkommen verletzungsfreie Thrombus-Materialablation von der Gefäßinnenwand, zumal bei Erreichen der gesunden Gefäßinnenwand durch den Katheter das gesunde Gefäßwandmaterial parallel zur Katheteraußenwand anliegt und nicht in die aufgeweiteten Wandöffnungen hineinzureichen vermag. Der mit seitlichen Wandöffnungen versetzte Katheter vermag somit lediglich Gefäßwand-adhärentes Gewebematerial abzutragen, das über die Gefäßwandoberfläche hinausragt.

Um wandständiges Thrombus-Material vollständig zu beseitigen, bedarf es zumeist einer mehrmaligen hintereinander durchzuführenden Positionierung des Katheters relativ zum intravasal vorhandenen Thrombus-Material und dem vorstehend beschriebenen Abtrennvorgang. Zwar ist es möglich, den Katheter nach jeder einzelnen Gewebeabtrennung durch einen ebenfalls intrakorporal eingeführten Arbeitskatheter proximalwärts zurückzuziehen und entsprechend extrakorporal von dem abgetrennten Gewebematerial zu säubern, um den Katheter erneut intrakorporal zu positionieren, doch ist es in Fällen, in denen größere Mengen an Thrombus-Material an einer Stenose abzutragen ist, vorteilhaft, proximalseits am Katheter eine Unterdruckquelle anzuschließen, über die abgetrenntes Thrombus-Material innerhalb des Katheters proximalseitig extrakorporal verbracht werden kann.

Weitere vorteilhafte Ausgestaltungsformen sind der weiteren Beschreibung unter Bezugnahme auf die Figuren zu entnehmen.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1a, b,c: Katheter und Mandrin im getrennten Zustand (a) sowie im gefügtem Zustand (b), Deatilansicht einer aufgeweiteten Wandöffnung,
- Fig. 2a,b,c: Sequenzbilddarstellungen zur Abtrennung von Gefäßwand-adhärentem weichen Thrombosematerial,
- Fig. 3: Ausführungsbeispiel eines Mandrins mit konkaven Ausnehmungen
- Fig. 4: Ausführungsbeispiel des Mandrins als Hohlmandrin mit Mandrinwandöffnungen,
- Fig. 5: Kathetersystem mit inflatierbarem Ballon sowie
- Fig. 6: Katheter mit Führungslumen im Bereich der Katheterspitze.

### Wege zur Ausführung der Erfindung, gewerbliche Verwendbarkeit

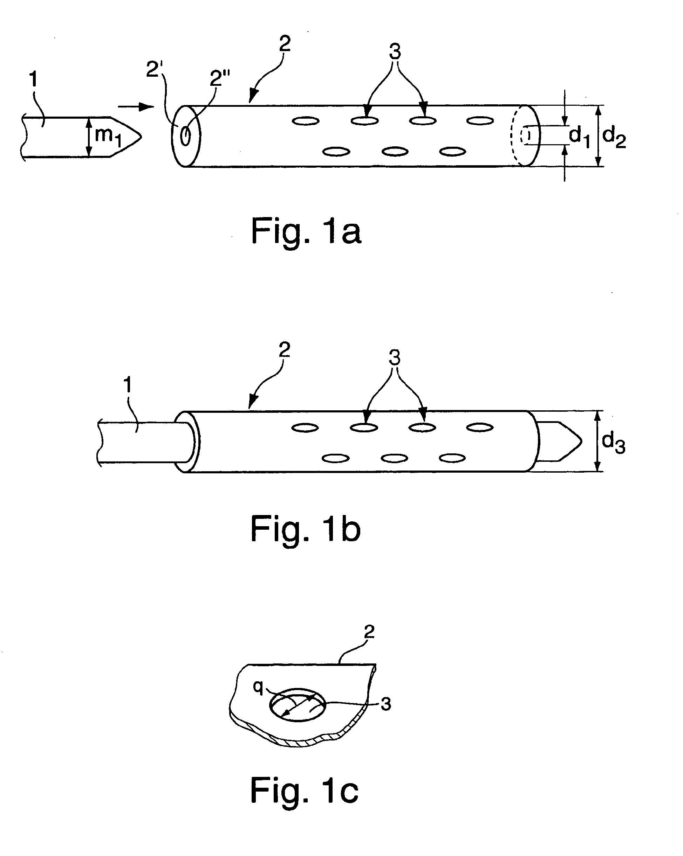

Fig. 1a zeigt einen Längsabschnitt des distalen Bereiches eines Katheters 2, der rohr- bzw. schlauchförmig ausgebildet ist und eine aus elastisch verformbaren Elastomermaterial bestehende Katheterwand 2' aufweist, die ein inneres Katheterlumen 2" umfasst. Das elastomere Katheterwandmaterial ist vorzugsweise aus einem Material der nachfolgenden Materialien gefertigt: Polymethylmethacrylat (PMMA), Polytetrafluoroethylen (PTFE), Polyurethane, Polyetherurethane, Silicon-Polyetherurethane, Silicon-Polyurethane, Silicon-Polycarbonat-Urethane, Polyolefin-Elastomere, Polylsobutylene, Fluorosilicone,Polyvinylchlorid (PVC), Polydimethylsiloxan (PDMS), Polylactide, Polyethylen,Polybutylmethacrylat, Polyacrylamid, Polyacrylonitrile, Polyamide, Polyetheramide,Polyethylenamin Polyimide, Polycarbonate, Polycarbourethane, Polyvinylketone, Polyvinylhalogenide, Polyvinylidenhalogenide, Polyvinylether, Polyisobutylene, Polyvinylaromaten, Polyvinylester, Polyvinylpyrollidone, Polyoxymethylene, Polytetramethylenoxid, Nylon oder Polyester.

Ferner sind in die Katheterwand Wandöffnungen 3 eingebracht, deren Anzahl, Anordnung, Öffnungsgröße und Öffnungsform grundsätzlich beliebig ausgestaltet sein können. Vorzugsweise weisen die Wandöffnungen 3 in dem in Fig. 1a gezeigten Zustand des Katheters, in dem der Katheter frei von äußeren Krafteinwirkungen ist, eine in Katheterlängserstreckung größere Öffnungsweite auf, als in Umfangsrichtung um den Katheter 2. Beispielsweise können die Wandöffnungen 3 durch lokale Einschnitte in Längsrichtung innerhalb der Katheterwand ausgeführt sein. Nicht notwendigerweise ist es erforderlich, die Form und Größe der Wandöffnungen 3 einheitlich vorzunehmen. Die Wandöffnungen 3 können durchaus je nach Anordnungsmuster längs und in Umfangsrichtung zum Katheter unterschiedliche Größen und Formen aufweisen.

Ferner ist in Fig. 1a ein stabförmig ausgebildeter Mandrin 1 dargestellt, der vorzugsweise aus einem Material gefertigt ist, das formstabil quer und flexibel zur Mandrinlängsachse ausgebildet ist. Beispielsweise eignen sich hierzu aus Kunststoff oder Metall gefertigte stab- oder drahtförmige Formkörper. Der Mandrindurchmesser m1 ist größer als der Innendurchmesser d1, vorzugsweise größer als der Katheteraußendurchmesser d2 des in Fig. 1 a illustrierten Katheters 2.

In Fig. 1 b ist ein Zustand illustriert, in dem der Mandrin 1 durch den Katheter 2 hindurchgeführt ist, wobei der Mandrin 1 den aus elastischem Material bestehenden Katheter 2 radial aufweitet. Der durch den mechanischen Zwang aufgeweitete Katheter 2 weist in diesem Zustand einen Außendurchmesser d3 auf, der größer ist als der Katheteraußendurchmesser d2 gemäß Fig. 1 a. Durch die zwangsweise radiale Aufweitung des Katheters 2 gemäß Fig. 1 b erfahren auch die Wandöffnungen 3 im Wege der elastischen Verformung der Katheterwand 2' eine zumindest in Umfangsrichtung um den Katheter 2 orientierte Öffnungsaufweitung. In Fig. 1c ist eine Detailansicht einer aufgeweiteten Wandöffnung 3 innerhalb der radial aufgespannten Katheterwand 2' gezeigt.

Zu Zwecken der intravasalen Positionierung in typischerweise blutdurchströmten Gefäßen, weist der in Fig. 1a illustrierte distale Katheterabschnitt vorzugsweise eine Länge von 1 cm bis 100 cm auf, wobei der Katheteraußendurchmesser d2 je nach medizinischer Applikation zwischen 0,5 mm und 25 mm misst. Die Wandstärke w des frei von äußeren Kräften vorliegenden Katheters 2 misst typischerweise zwischen 0,1 mm und 2,5 mm.

Als besonders vorteilhaft hat es sich für den lokalen Abtrag von weichem, Gefäßwand-adhärentem Thrombus-Material erwiesen, die Wandöffnungen 3 derart zu dimensionieren, so dass die Wandöffnungen 3 im aufgeweiteten Zustand (siehe Fig. 1b und 1 c) eine größte Öffnungsweite q von 0,1 mm bis 15 mm besitzt.

Aufgrund der elastomeren Materialwahl für die Katheterwand 2' liegt es auf der Hand, dass sich nach Entfernen des Mandrins 1 aus dem Katheter 2 der Katheter aufgrund Material-inhärenter elastischer Rückstellkräfte selbständig und unverzüglich in den Zustand mit dem kleineren Außendurchmesser d2 rückverformt, wodurch zugleich auch die aufgeweiteten Wandöffnungen 3 die ursprüngliche kleine Wandöffnungsgeometrie annehmen. Eben diesen selbständigen Rückverformungsvorgang, der auf den Material-inhärenten elastischen Rückstellkräften innerhalb des Katheters beruht, macht sich das lösungsgemäße Kathetersystem zunutze, um einen schonenden Gewebeabtrag innerhalb eines Gefäßes zu realisieren.

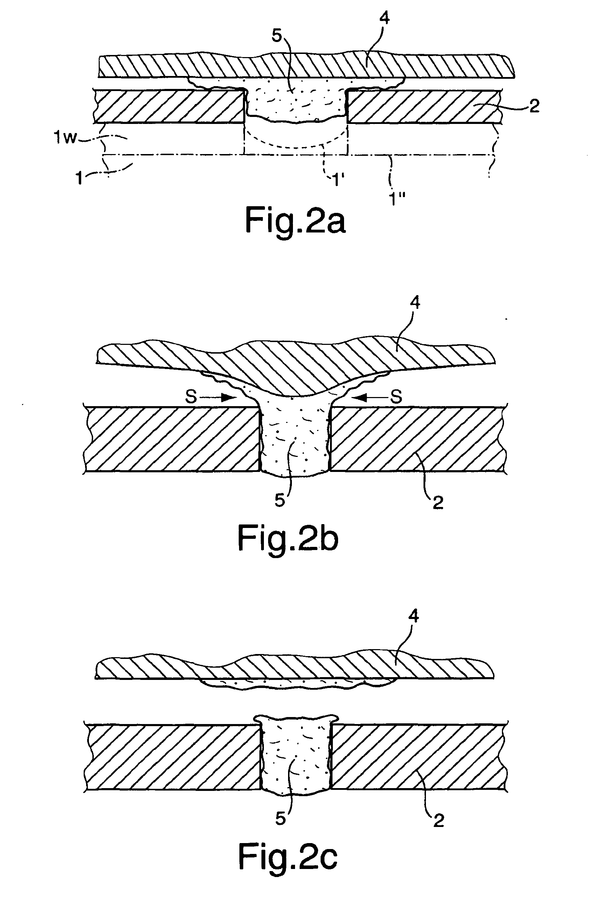

In den folgenden Sequenzbildern gemäß der Figuren 2a bis 2c sei dieser schonende Gewebeabtrag schematisch illustriert.

Fig. 2a zeigt einen Teilquerschnitt durch einen intravasal relativ zu einem an einer Gefäßwand 4 adhärenten Thrombus 5 positionierten Katheter 2 im radial aufgeweiteten Zustand, bei dem innerhalb des Katheters 2 der Mandrin 1 eingeführt ist. Die in Fig. 2a im Querschnitt dargestellte Wandöffnung 3 ist aufgeweitet und bietet die Möglichkeit, dass ein Teil des Gefäßwand-adhärenten Thrombus-Materials 5 in die Öffnungsweite der Wandöffnung 3 hineinragt. Durch die radiale Aufweitung des Katheters 2 ist zudem dafür gesorgt, dass die Katheteraußenwand 2 mit einem durch die Geometriewahl von Mandrin und Katheter vorgebbaren Anpressdruck gegen das Gefäßwand-ständige Thrombus-Material 5 angepresst wird, wodurch sichergestellt ist, dass innerhalb der wandaufgeweiteten Wandöffnung 3 Thrombus-Material hineinreicht.

Wird im nächsten Schritt der Mandrin 1 proximalwärts aus dem Katheterabschnitt entfernt, so wirken die vorstehend beschriebenen elastischen Rückstellkräfte und bewirken ein Zusammenziehen bzw. Verkleinern der Wandöffnung 3, wodurch das innerhalb der Wandöffnung 3 befindliche Thrombus-Material 5 regelrecht eingequetscht wird (siehe hierzu Fig. 2b).

Durch die elastische Rückformung des Katheters 2 entstehen Scherkräfte S, die dafür sorgen, dass das von dem Katheter 2 erfasste Thrombus-Material 5 vom an der Gefäßwand 4 verbleibenden Restmaterial abgeschert und separiert wird. Fig. 2c zeigt den Zustand des Katheters 2 mit von der Gefäßwand 4 abgetrenntem Thrombus-Material 5, das innerhalb der eingeengten Wandöffnung 3 sicher längs des Katheters 2 fixiert bleibt, um auf diese Weise das abgetrennte Thrombus-Material proximalwärts zu entfernen.

In vorteilhafter Weise kann der in den Fig. 2a bis 2c illustrierte Abtrennvorgang zusätzlich unterstützt werden durch die Applikation eines längs des Katheters 2 anliegenden Unterdrucks.

Eine weitere vorteilhafte Ausbildungsvariante für die Ausgestaltung des Mandrins 1 ist zusätzlich in Fig. 2a illustriert. Die strichliert angedeutete Oberflächenkontur 1' illustriert eine lokale konkave Ausnehmung innerhalb der Mandrinoberfläche, die gegenüber der aufgeweiteten Wandöffnung 3 innerhalb des Katheters 2 angeordnet ist. Durch eine derartige Ausnehmung 1' kann das Gewebeentnahmevolumen gegenüber einer glatt ausgebildeten Mandrinoberfläche vergrößert werden.

Ferner ist in Figur 2a durch die strichpunktierten Linien 1" eine weitere Ausbildungsvariante des Mandrins 1 als Hohlmandrin 1" angedeutet, der in der Hohlmandrinwand 1w wenigstens eine Mandrindwandöffnung 1"' aufweist. Durch die Mandrinwandöffnung 1'" ist zum einen das Gewebeentnahmevolumen nochmals deutlich vergrößert im Vergleich zu den beiden vorstehend beschriebenen Alternativen, zum anderen ist es möglich längs des Hohlmandrins Unterdruck anzulegen, durch den die Thrombus-Material Entnahme sowie Separierung signifikant unterstütz werden kann.

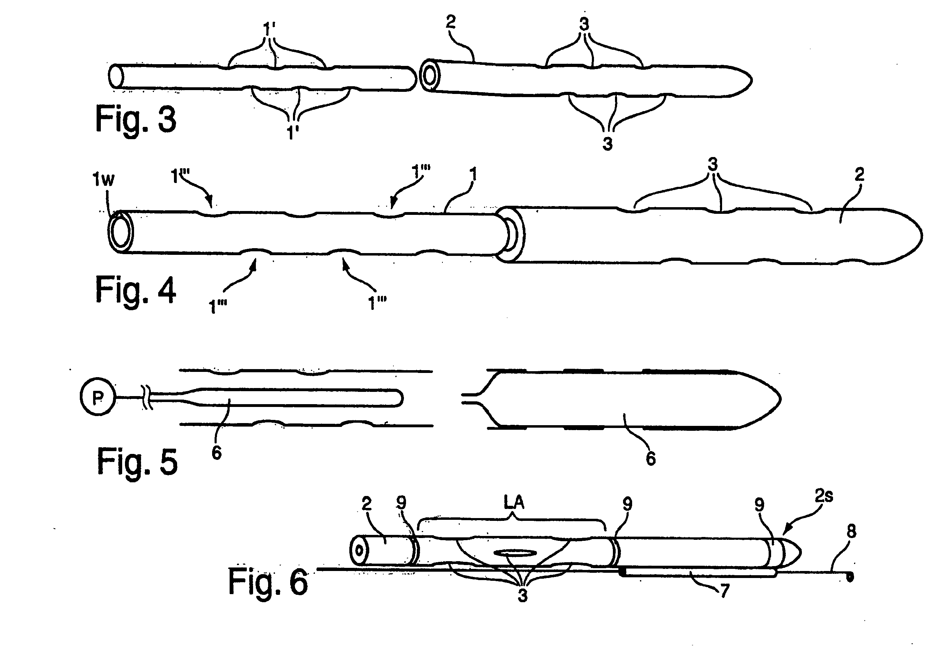

Fig. 3 zeigt einen entsprechend konfektionierten Mandrin 1, an dessen Mandrinoberfläche lokale konkave Ausnehmungen 1' eingebracht sind, die in Größe und Anordnung mit den innerhalb des Katheters 2 eingebrachten Wandöffnungen 3 im radial aufgeweiteten Zustand übereinstimmen bzw. weitgehend übereinstimmen. Der Katheter 2 verfügt typischerweise über eine distale Katheterspitze 2s.

Fig. 4 zeigt einen Hohlmandrin mit Mandrinwandöffnungen 1'" in der Mandrinwand 1w, die in Größe und Anordnung mit den innerhalb des Katheters 2 eingebrachten Wandöffnungen 3 im radial aufgeweiteten Zustand übereinstimmen bzw. weitgehend übereinstimmen.

Fig. 5 zeigt eine Ausbildungsalternative mit einem axial längs des Katheters 2 einführbaren inflatierbaren Ballon 6, der proximalwärts mit einer Pumpvorrichtung P verbunden ist. Der Ballon 6 übt im deflatierten Zustand keine radial auf den Katheter 2 wirkenden Kräfte aus (siehe rechte Darstellung in Fig. 5). Wird hingegen der Ballon 6 inflatiert (siehe linke Darstellung), so üben die pneumatischen Kräfte eine radial orientierte elastische Verformarbeit auf den Katheter 2 aus, wodurch dieser den vorstehend erläuterten radial aufgeweiteten Zustand annimmt. Durch entsprechende Deflation des Ballons 6 gelangt der Katheter 2 selbständig wieder in seine Ursprungsform.

In Figur 6 ist ein Teil des Katheters 2 abgebildet, längs dessen Erstreckung der mit der wenigstens einen Wandöffnung 3 versehene Längsabschnitt LA vorgesehen ist, der proximalseitig zur Katheterspitze 2s beabstandet ist. Zur intrakorporalen Positionierung des Katheters 2 und insbesondere dessen Längsabschnitt LA relativ zu Gefäßwandständigem Thrombusmaterial sieht der Katheter 2 zwischen dem Katheterlängsabschnitt LA und der Katheterspitze 2s ein zusätzliches Führungslumen 7 vor, das im dargestellten Fall an der Aussenwand des Katheters 2 angebracht ist, durch das ein Führungsdraht 8 hindurchführbar ist. Selbstverständlich ist das Führungslumen 7 vorzugsweise integral innerhalb des Katheters 2 vorzusehen. Die Länge des Führungslumens 7 misst typischerweise 20 bis 50 mm.

Um den Positioniervorgang des Katheters relativ zu einem mit Thrombus-Material befallenen Gefäßbereich für den Operateur überwachen zu können, sind längs des Katheters 2, insbesondere an der Katheterspitze 2s und/oder im Bereich des Katheterlängsabschnittes LA röntgendichte Markierungen 9 angebracht, die auf online Röntgenaufnahmen sichtbar hervortreten.

Alle beschriebenen Kathetervarianten verfügen über den Katheter 2, dessen Katheterspitze 2s vorzugsweise geschlossen ausgebildet ist. Selbstverständlich sind auch offen ausgebildete Katheterspitzen 2s denkbar, um auf diese Weise bspw. distalseitig unmittelbar vor der Katheterspitze 2s befindliches Thrombus-Material bspw. mittels Unterdruck abzutragen.

Das vorstehend beschriebene Kathetersystem zeichnet sich durch seinen einfachen Komponentenaufbau aus und gewährleistet einen sicheren und schonenden Abtrag von intravaskulären, weichen Gewebeablagerungen. Das lösungsgemäße Kathetersystem ist für sämtliche Bereiche der intravaskulären Gefäßmedizin anwendbar, so insbesondere für die interventionelle Radiologie, Angiologie, Kardiologie oder Herz- und Gefäßchirurgie.

### Bezugszeichenliste

- 1: Mandrin
- 1': Konkave Ausnehmungen
- 1": Strichpunktierte Linie, Hohlmandrin
- 1"': Mandrinwandöffnung
- 1w: Mandrinwand
- 2: Katheter
- 2': Katheterwand
- 2": Katheterlumen
- 3: Wandöffnung
- 4: Gefäßwand
- 5: Thrombus-Material
- 6: Ballon
- 1': Konkave Ausnehmung
- d1: Katheterinnendurchmesser im kräftefreien Zustand
- d2: Katheteraußendurchmesser im kräftefreien Zustand
- d3: Katheteraußendurchmesser im radial aufgeweiteten Zustand
- m1: Mandrindurchmesser
- q: Wandöffnungsdurchmesser im aufgeweiteten Zustand
- P: Pumpvorrichtung

## Patentansprüche

1. Vorrichtung zum Ablösen wandständiger Thromben aus einem Körpergefäß mit einem Katheter (2), längs dessen Katheterlängserstreckung wenigstens ein Katheterabschnitt vorgesehen ist, in dessen zuordenbaren Katheterwand (2') wenigstens eine die Katheterwand (2') quer zur Katheterlängserstreckung vollständig durchsetzende Wandöffnung (3) vorgesehen ist, sowie einem Mittel, mit dem ein dem Katheterabschnitt zuordenbarer Katheteraußendurchmesser veränderbar ist, **dadurch gekennzeichnet, dass** die Katheterwand (2') längs des wenigstens einen Katheterabschnitts aus einem elastisch verformbaren Material besteht,
dass der Katheter (2) durch das, einen auf den Katheter (2) wirkenden äußeren Zwang verursachende Mittel von einem Zustand mit relativ kleinerem Katheteraußendurchmesser (d2) in einen Zustand mit relativ größerem Katheteraußendurchmesser (d3) im Wege einer elastischen Formänderung überführbar ist, wobei das Mittel als ein in den wenigstens einen Katheterabschnitt einführbarer Verdrängungskörper ausgebildet ist,
dass unter Wegfall des äußeren Zwangs der Katheter (2) durch materialinhärente, elastische Rückstellkräfte den Zustand mit dem relativ kleineren Katheteraußendurchmesser (d2) selbständig einnimmt.

2. Vorrichtung nach Anspruch 1,
**dadurch gekennzeichnet, dass** die Katheterwand (2') des Katheterabschnittes rohrförmig ausgebildet ist, ein inneres Katheterlumen (2") umfasst und das elastisch verformbare Material ein Elastomer ist.

3. Vorrichtung nach Anspruch 2,
**dadurch gekennzeichnet, dass** das Elastomer aus wenigstens einem Material aus den nachfolgenden Materialien besteht:
Polymethylmethacrylat (PMMA), Polytetrafluoroethylen (PTFE), Polyurethane, Polyetherurethane, Silicon-Polyetherurethane, Silicon-Polyurethane, Silicon-Polycarbonat-Urethane, Polyolefin-Elastomere, Polylsobutylene, Fluorosilicone,Polyvinylchlorid (PVC), Polydimethylsiloxan (PDMS), Polylactide, Polyethylen,Polybutylmethacrylat, Polyacrylamid, Polyacrylonitrile, Polyamide, Polyetheramide,Polyethylenamin Polyimide, Polycarbonate, Polycarbourethane, Polyvinylketone, Polyvinylhalogenide, Polyvinylidenhalogenide, Polyvinylether, Polyisobutylene, Polyvinylaromaten, Polyvinylester, Polyvinylpyrollidone, Polyoxymethylene, Polytetramethylenoxid, Nylon oder Polyester.

4. Vorrichtung nach einem der Ansprüche 1 bis 3,
**dadurch gekennzeichnet, dass** der Katheterabschnitt in Katheterlängserstreckung 1 cm bis 100 cm lang ausgebildet ist, einen elastischen Abschnitt mit einem Katheteraußendurchmesser (d2) von 1 mm bis 25 mm sowie eine Katheterwandstärke ohne Einwirkung eines äußeren Zwangs zwischen 0,1 mm und 2,5 mm aufweist.

5. Vorrichtung nach einem der Ansprüche 1 bis 4,
**dadurch gekennzeichnet, dass** die wenigstens eine Wandöffnung (3) im Zustand mit relativ größerem Katheteraußendurchmesser (d3) eine größte Öffnungsweite (q) von 0,1 mm bis 15 mm aufweist.

6. Vorrichtung nach einem der Ansprüche 1 bis 5,
**dadurch gekennzeichnet, dass** längs des Katheterabschnittes eine Vielzahl von Wandöffnungen (3) vorgesehen ist, die sowohl längs als auch in Umfangsrichtung des Katheterabschnittes gleich verteilt angeordnet ist.

7. Vorrichtung nach einem der Ansprüche 1 bis 6 ,
**dadurch gekennzeichnet, dass** das Mittel als stab- oder drahtartiger Mandrin (1) ausgebildet ist, der in Längsrichtung flexibel und quer zur Längsrichtung formstabil ausgebildet ist und in Abhängigkeit von Form und Größe des Katheters (2) derart dimensioniert ist, dass durch ein Einführen des Mandrins (1) in den Katheter (2) der Katheterabschnitt von dem Zustand mit relativ kleinerem Katheteraußendurchmesser (d2) in den Zustand mit relativ größerem Katheteraußendurchmesser (d3) überführbar ist.

8. Vorrichtung nach Anspruch 7,
**dadurch gekennzeichnet, dass** der Mandrin (1) eine ansonsten glatte Oberfläche besitzt, die über wenigstens eine konkave Vertiefung oder Ausnehmung (1') verfügt, und dass die wenigstens eine konkave Vertiefung oder Ausnehmung (1') derart längs der Oberfläche des Mandrins verteilt angeordnet ist, dass im Zustand des in den Katheter (2) eingeführten Mandrins (1) die wenigstens eine konkave Vertiefung oder Ausnehmung (1') mit der wenigstens einen Wandöffnung (3) radial überlappt.

9. Vorrichtung nach Anspruch 7,
**dadurch gekennzeichnet, dass** der Mandrin (1) als Hohlmandrin (1") ausgebildet ist und eine ansonsten glatte Oberfläche besitzt, die über wenigstens eine Mandrinwandöffnung (1"') verfügt, und dass die wenigstens Mandrinwandöffnung (1"') derart längs der Oberfläche des Mandrins (1) angeordnet ist, dass im Zustand des in den Katheter (2) eingeführten Hohlmandrins (1 ") die wenigstens eine Mandrinwandöffnung (1"') mit der wenigstens einen Wandöffnung (3) radial überlappt.

10. Vorrichtung nach einem der Ansprüche 1 bis 6,
**dadurch gekennzeichnet, dass** das Mittel ein inflatierbarer Ballon (6) ist, der in Abhängigkeit von Form und Größe des Katheters (2) derart dimensioniert ist, dass der Ballon (6) in den Katheter (2) einführbar ist und durch Inflatieren des Ballons (2) den Katheterabschnitt von dem Zustand mit relativ kleinerem Katheteraußendurchmesser (d2) in den Zustand mit relativ größerem Katheteraußendurchmesser (d3) überführt.

11. Vorrichtung nach einem der Ansprüche 1 bis 10,
**dadurch gekennzeichnet, dass** die Katheterwand (2') längs des wenigstens einen Katheterabschnitts eine Wandaußenseite aufweist, die über hydrophilie Eigenschaften verfügt.

12. Vorrichtung nach einem der Ansprüche 1 bis 11,
**dadurch gekennzeichnet, dass** der Katheter (2) proximalseitig einen Anschluss vorsieht, an den eine Unterdruckquelle (P) und/oder eine motorische Vibrationseinheit anschließbar ist/sind.

13. Vorrichtung nach einem der Ansprüche 1 bis 12,
**dadurch gekennzeichnet, dass** im Bereich der dem Katheter (2) zuordenbaren Katheterspitze und/oder längs des elastischen Katheterabschnittes wenigstens eine röntgendichte Markierung angebracht ist.

14. Vorrichtung nach einem der Ansprüche 1 bis 13,
**dadurch gekennzeichnet, dass** von der dem Katheter (2) zuordenbaren Katheterspitze bis zum elastischen Katheterabschnitt an der Katheteraußenseite über eine Länge von 20-50 mm ein weiteres Lumen zur Führung des Katheters (2) über einen Führungsdraht angebracht ist.

15. Vorrichtung nach einem der Ansprüche 1 bis 14,
**dadurch gekennzeichnet, dass** die wenigstens eine Wandöffnung (3) einen schlitzförmigen, runden, ovalen oder n-eckigen Öffnungsquerschnitt aufweist, der sich beim Überführen des Katheters (2) vom Zustand mit dem relativ kleineren Katheteraußendurchmesser (d2) in den Zustand mit dem relativ größeren Katheteraußendurchmesser (d3) durch den äußeren Zwang aufweitet und im umgekehrten Fall selbständig verkleinert.

## Claims

1. Device for the detachment of parietal thrombi from a body vessel with a catheter (2), along the longitudinal extension of which at least one catheter section is provided, in the assignable catheter wall (2') of which at least one wall opening (3) is provided which passes completely through the catheter wall (2') perpendicularly to the longitudinal extension of the catheter, as well as a means with which a external catheter dimension assignable to the catheter section can be adjusted, **characterised in that** the catheter wall (2') along the at least one catheter section is made of an elastically deformable material, **in that** through the means exerting an external force on the catheter (2), the catheter (2) can be changed from a state with a relatively smaller external catheter diameter (d2) into a state with a relatively larger external catheter diameter (d3) by way of an elastic change in shape, wherein the means is designed as a displacement body that can be introduced into the at least one catheter section, **in that** on removal of the external force, through material-inherent elastic resorting forces, the catheter (2) assumes the state with the relatively smaller diameter (d2) by itself.

2. Device according to claim 1 **characterised in that** the catheter wall (2') of the catheter section tubular in design, comprises an inner catheter lumen (2'') and the elastically deformable material is an elastomer.

3. Device according to claim 2, **characterised in that** the elastomer comprises at least one material out of the following materials:
polymethyl methacrylate (PMMA), polytetrafluoroethylene (PTFE), polyurethanes, polyether urethanes, silicone polyether urethanes, silicone polyurethanes, silicone polycarbonate urethanes, polyolefin elastomers, polyisobutylene fluorosilicones, polyvinyl chloride (PTC), polydimethylsiloxane (PDMS), polyactides, polyethylene, polybutyl methacrylate, polyacrylamide, polyacrylonitrile, polyamides, polycarbonates, polycarbourethanes, polyvinyl ketones, polyvinylhalogenides, polyvinylidene halogenides, polyvinylether, polyisobutylenes, polyvinyl aromates, polyvinylester, polyvinyl pyrollidiones, polyoxymethylenes, polytetramethyl oxide, nylon on polyester.

4. Device according to claims 1 to 3 **characterised in that** the catheter section in the longitudinal extension of catheter is 1 cm to 100 cm in length and has an elastic section with external catheter diameter (d2) of 1 mm to 25 mm as well as a catheter wall thickness of between 0.1 mm and 2.5 mm without the effect of the external force.

5. Device according to any one of claims 1 to 4 **characterised in that** the at least one wall opening (3) in the state with a relatively larger external catheter dimension (d3) has a largest opening width (q) of 0.1 mm to 15 mm.

6. Device according to any one of claims 1 to 5 **characterised in that** along the catheter section a plurality of wall openings (3) are provided, which both longitudinally and in the circumferential direction of the catheter section are arranged in an equally distributed manner.

7. Device according to any one of claims 1 to 6 **characterised in that** the means is designed in the form of a rod or wire-like mandrel (1) which in the longitudinal direction is flexible and is dimensionally stable perpendicularly to the longitudinal direction and, depending on the shape and size of the catheter (2) is dimensioned in such a way that through introducing the mandrel (1) into the catheter (2), the catheter section can be changed from the state with the relatively smaller external catheter diameter (d2) into the state with the relatively larger external catheter diameter (d3).

8. Device according to claim 7 **characterised in that** the mandrel (1) has an otherwise smooth surface which has at least one concave indentation or recess (1') and that the at least one concave indentation or recess (1') is arranged distributed along the surface of the mandrel in such a way that in the state in which the mandrel (1) is inserted into the catheter (2), the at least one concave indentation or recess (1') radially overlaps with the at least one wall opening (3).

9. Device according to claim 7 **characterised in that** the mandrel (1) is designed as a hollow mandrel (1'') and has an otherwise smooth surface which has at least one mandrel wall opening (1''') and **in that** the at least one mandrel wall opening (1''') is arranged along the surface of the mandrel (1) in such a way that in the state in which the hollow mandrel (1'') is inserted into the catheter (2) the at least one mandrel wall opening (1''') overlaps at least one wall opening (3).

10. Device according to any one of claims 1 to 6 **characterised in that** the means is an inflatable balloon (6), which, depending on the shape and size of the catheter (2) is dimensioned in such a way that the balloon (6) can be inserted into the catheter (2) and through inflation of the balloon (2) changes the catheter section from the state with the relatively smaller external catheter diameter (d2) into the state with the relatively larger external catheter diameter (d3).

11. Device according to any one of claims 1 to 10 **characterised in that** along the at least one catheter section, the catheter wall (2') has an external wall side which possesses hydrophilic properties.

12. Device according to any one claims 1 to 11 **characterised in that** on the proximal side the catheter (2) envisages a connection to which a negative pressure source (P) and/or a motor vibration unit can be connected.

13. Device according to any one of claims 1 to 12 **characterised in that** in the area of the catheter tip which can be assigned to the catheter (2) and/or along the elastic catheter section at least one radio-opaque marking in applied.

14. Device according to any one of claims 1 to 13 **characterised in that** from the catheter tip assignable to the catheter (2) to the elastic catheter section on the outer side of the catheter over a length of 20 mm - 50 mm a further lumen is applied for guiding the catheter (2) via a guide wire.

15. Device according to any one of claims 1 to 14 **characterised in that** the at least one wall opening (3) has a slit-like, round, oval or n-cornered opening cross-section, which when the catheter (2) is changed from the state with the relatively smaller external catheter diameter (d2) into the state with the relatively larger external catheter diameter (d3) widens through the external force and in the opposite case become smaller by itself.

## Revendications

1. Dispositif pour enlever des thrombus muraux d'un canal corporel comprenant un cathéter (2) le long de l'extension longitudinale duquel est prévu au moins un tronçon de cathéter, au moins une ouverture de paroi (3) traversant intégralement la paroi de cathéter (2') transversalement à l'extension longitudinale de cathéter est prévue dans sa paroi de cathéter (2) correspondante, ainsi qu'un moyen avec lequel un diamètre extérieur de cathéter correspondant au tronçon de cathéter est modifiable, **caractérisé en ce que** la paroi de cathéter (2') est composée d'un matériau à déformation élastique le long de l'au moins un tronçon de cathéter, que le cathéter (2) peut passer d'un état avec un diamètre extérieur de cathéter (d2) relativement plus petit à un état avec un diamètre extérieur de cathéter (d3) relativement plus grand dans le trajet d'une modification de forme élastique, par le moyen provoquant une contrainte extérieure agissant sur le cathéter (2), sachant que le moyen est conçu en tant que corps de poussée, qu'en cas d'absence de contrainte extérieure, le cathéter (2) adopte l'état avec le diamètre extérieur de cathéter (d2) relativement plus petit par des forces de rappel élastiques inhérentes au matériau.

2. Dispositif selon la revendication 1, **caractérisé en ce que** la paroi de cathéter (2') du tronçon de cathéter est tubulaire, comprend une lumière de cathéter interne (2'') et que le matériau à déformation élastique est un élastomère.

3. Dispositif selon la revendication 2, **caractérisé en ce que** l'élastomère est composé d'au moins un matériau des matériaux suivantes :
polyméthylméthacrylate (PMMA), polytétrafluoroéthylène (PTFE), polyuréthane, polyétheruréthane, silicone-polyétheruréthane, silicone-polyuréthane, silicone-polycarbonate-uréthane, polyoléfine-élastomère, polylsobutylène, fluorosilicone, chlorure de polyvinyle (PVC), polydiméthylsiloxane (PDMS), polyactide, polyéthylène, polybutylméthacrylate, polyacrylamide, polyacrylonitrile, polyamide, polyétheramide, polyéthylènamine, polyimide, polycarbonate, polycarbo-uréthane, polyvinyle cétone, halogénure de polyvinyle, halogénure de polyvinylidène, polyvinyléther, polyisobutylène, aromates de polyvinyle, ester de polyvinyle, pyrollidone (polyvinyle), polyoxyméthylène, oxyde de polytétraméthyle, nylon ou polyester.

4. Dispositif selon l'une des revendications 1 à 3, **caractérisé en ce que** le tronçon de cathéter dans l'extension longitudinale de cathéter est conçu avec une longueur de 1 cm à 100 cm, présente un tronçon élastique avec un diamètre extérieur de cathéter (d2) de 1 mm à 25 mm ainsi qu'une épaisseur de paroi de cathéter entre 0,1 mm et 2,5 mm sans effet d'une contrainte extérieure.

5. Dispositif selon l'une des revendications 1 à 4, **caractérisé en ce que** l'au moins une ouverture de paroi (3) présente une largeur d'ouverture la plus grande (q) de 0,1 mm à 15 mm dans l'état avec le diamètre extérieur de cathéter (d3) relativement plus grand.

6. Dispositif selon l'une des revendications 1 à 5, **caractérisé en ce qu'**une pluralité d'ouvertures de paroi (3) est prévue le long du tronçon de cathéter, qui sont réparties uniformément tant dans le sens longitudinal que périphérique du tronçon de cathéter.

7. Dispositif selon l'une des revendications 1 à 6, **caractérisé en ce que** le moyen est conçu en tant que mandrin (1) de type tige ou fil qui est conçu flexible dans le sens longitudinal et à forme stable transversalement au sens longitudinal, et est dimensionné de telle façon en fonction de la forme et de la taille du cathéter (2) que, par une introduction du mandrin (1) dans le cathéter (2), le tronçon de cathéter peut passer de l'état avec le diamètre extérieur de cathéter (d2) relativement plus petit à l'état avec le diamètre extérieur de cathéter (d3) relativement plus grand.

8. Dispositif selon la revendication 7, **caractérisé en ce que** le mandrin (7) possède sinon une surface lisse qui dispose d'au moins un creux ou évidement concave (1'), et que l'au moins un creux ou évidement concave (1') est ainsi réparti le long de la surface du mandrin que, dans l'état où le mandrin (1) est introduit dans le cathéter (2), l'au moins un creux ou évidement concave (1') se chevauche radialement avec l'au moins une ouverture de paroi (3).

9. Dispositif selon la revendication 7, **caractérisé en ce que** le mandrin (7) est conçu en tant que mandrin creux (1'') et possède sinon une surface lisse qui dispose d'au moins une ouverture de paroi de mandrin (1'''), et que l'au moins une ouverture de paroi de mandrin (1''') est disposée de telle sorte le long de la surface du mandrin (1) que dans l'état où le mandrin (1) est introduit dans le cathéter (2), l'au moins une ouverture de paroi de mandrin (1''') se chevauche radialement avec l'au moins une ouverture de paroi (3).

10. Dispositif selon l'une des revendications 1 à 6, **caractérisé en ce que** le moyen est un ballon gonflable (6) qui est ainsi dimensionné en fonction de la forme et de la taille du cathéter (2) que le ballon (6) peut être introduit dans le cathéter (2) et que quand le ballon gonfle (2), le tronçon de cathéter passe de l'état avec le diamètre extérieur de cathéter (d2) relativement plus petit à l'état avec le diamètre extérieur de cathéter (d3) relativement plus grand.

11. Dispositif selon l'une des revendications 1 à 10, **caractérisé en ce que** la paroi de cathéter (2') présente une face extérieure de paroi qui a des propriétés hydrophiles le long de l'au moins un tronçon de cathéter.

12. Dispositif selon l'une des revendications 1 à 11, **caractérisé en ce que** le cathéter (2) prévoit un raccord côté proximal sur lequel une source de dépression (P) et/ou une unité vibrante motorisée peu(ven)t être raccordée(s).

13. Dispositif selon l'une des revendications 1 à 12, **caractérisé en ce qu'**au moins un marquage opaque aux rayons est apposé au niveau de la pointe de cathéter pouvant être attribuée au cathéter (2) et/ou le long du tronçon de cathéter élastique.

14. Dispositif selon l'une des revendications 1 à 13, **caractérisé en ce que** de la pointe de cathéter pouvant être attribuée au cathéter (12) au tronçon de cathéter élastique, une autre lumière pour guider le cathéter (2) est appliquée par un fil de guidage sur le côté extérieur de cathéter, sur une longueur de 20 mm - 50 mm.

15. Dispositif selon l'une des revendications 1 à 14, **caractérisé en ce que** l'au moins une ouverture de paroi (3) présente une section transversale d'ouverture fendue, ronde, ovale ou à n-angles, qui s'élargit par la contrainte forcée, lors du passage du cathéter (2) de l'état avec le diamètre extérieur de cathéter (d2) relativement plus petit à l'état avec le diamètre extérieur de cathéter (d3) relativement plus grand, et se réduit dans le cas inverse.
